(19) Europäisches Patentamt
European Patent Office
Office européen des brevets

(11) **EP 2 423 187 A1**

(12) **EUROPEAN PATENT APPLICATION**
published in accordance with Art. 153(4) EPC

(43) Date of publication:
**29.02.2012 Bulletin 2012/09**

(51) Int Cl.:
*C07C 269/04* (2006.01)    *C07C 271/12* (2006.01)

(21) Application number: **09843676.9**

(22) Date of filing: **13.07.2009**

(86) International application number:
**PCT/JP2009/062687**

(87) International publication number:
**WO 2010/122682 (28.10.2010 Gazette 2010/43)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.04.2009 JP 2009106185**

(71) Applicant: **Kaneka Corporation
Osaka-shi, Osaka 530-8288 (JP)**

(72) Inventors:
• **KAWASAKI, Hiroaki
Takasago-shi
Hyogo 676-8688 (JP)**

• **MOROSHIMA, Tadashi
Takasago-shi
Hyogo 676-8688 (JP)**
• **MAEHARA, Katsuji
Takasago-shi
Hyogo 676-8688 (JP)**
• **HIRAI, Yoshinori
Takasago-shi
Hyogo 676-8688 (JP)**

(74) Representative: **HOFFMANN EITLE
Patent- und Rechtsanwälte
Arabellastraße 4
81925 München (DE)**

(54) **PROCESS FOR PRODUCTION OF N-ALKOXYCARBONYL-tert-LEUCINES**

(57)    A high quality N-alcoxycarbonyl-tert leucine can be efficiently and stably produced with an easy procedure by reacting tert-leucine with an N-alcoxycarbonylating agent in the presence of water, wherein the use amount of the N-alcoxycarbonylating agent is not less than 0.90 times by mole and not more than 1.00 times by mole relative to the tert-leucine, with maintaining the pH of the reaction mixture in the range of not less than 9 and not more than 13 using a basic agent. In addition, an N-alcoxycarbonyl-tert-leucine can be efficiently extracted from the basic aqueous solution thereof under a mild condition using a water-immiscible solvent by mixing a hydroxide. Furthermore, the two crystal forms of N-butoxycarbonyl-tert-leucine can be controlled by adjusting the water amount in crystallization step, and the compound can be stably produced in an industrial production.

EP 2 423 187 A1

**Description**

TECHNICAL FIELD

The present invention relates to a process for production of an N-alcoxycarbonyl-tert-leucine, which is useful as an intermediate of a medicine or an agrichemical.

BACKGROUND ART

An N-alcoxycarbonyl-L-tert-leucine is obtained by alcoxycarbonylating the amino group of L-tert-leucine (2S-amino-3,3-dimethylbutanoic acid), and is useful as an intermediate of various medicines or agrichemicals. In particular, it is reported that N-tert-butoxycarbonyl-L-tert-leucine is very useful as a raw material of a hepatitis C virus protease inhibitor (Patent Documents 1 and 2).

As a process for production of an N-alcoxycarbonyl-tert-leucine, the following processes are known:

1) Production process of an N-alcoxycarbonyl-tert-leucine using an excessive N-alcoxycarbonylating agent with maintaining the pH between 11 and 13 (Patent Document 3);
2) Production process of an N-alcoxycarbonyl-tert-leucine by adding an excessive amount of sodium hydroxide in advance to obtain a strongly basic aqueous solution of tert-leucine and then adding an equimolar amount of an N-alcoxycarbonylating agent to the solution (Patent Document 4).

In addition, Patent Documents 5 and 6 disclose the process for production of an N-tert-butoxycarbonyl-L-tert-leucine by mixing di-tert-butyl dicarbonate with L-tert-leucine.

PRIOR ART

PATENT DOCUMENT

Patent Document 1 : WO2009/008913
Patent Document 2 : WO2009/005677
Patent Document 3 : JP2004-175703A
Patent Document 4 : JP2001-501216T
Patent Document 5 : JP2003-146962A
Patent Document 6 : JP2008-125364A

DISCLOSURE OF THE INVENTION

PROBLEMS TO BE SOLVED BY THE INVENTION

An N-alcoxycarbonylating reaction proceeds in a basic condition; however, the pH of the reaction mixture is generally decreased, since the amino group is protected and an acidic compound such as hydrochloric acid and a carbonic acid are produced as by-product with the reaction progress. It is therefore needed that the pH of the reaction mixture is adjusted (controlled) using a base in order to keep the reactivity of the amino group.

As the process for production of an N-alcoxycarbonyl-tert-leucine with adjusting the pH, for example, the process described in Patent Document 3 is known. However, it is needed in the process of Patent Document 3 that the pH has to be adjusted within a narrow range between 11 and 13. If the pH falls outside the specified range, yield and quality are considered to be significantly decreased.

However, it is not easy to maintain the pH within such a narrow range; therefore, it is also difficult to constantly produce high quality product. Such a difficulty has to be overcome especially in the field of medicine or agrichemical,

On the other hand, the process described in Patent Document 4 for producing an N-alcoxycarbonyl-tert-leucine can be easily carried out. In the process, an equimolar amount of an N-alcoxycarbonylating agent is used with maintaining the pH of the reaction mixture at a strongly basic condition by adding an excessive amount of a base in advance. However, the present inventors found that the process is not necessarily efficient, since an N-alcoxycarbonylating agent is decomposed due to a strongly basic condition, as a result, the reaction cannot be completed and the yield is decreased.

In Patent Document 5, with respect to the extraction after the reaction, a low polarity solvent such as an aromatic hydrocarbon solvent is used in terms of easy extraction procedure when N-tert-butoxycarbonyl-L-tert-leucine is extracted from the solution thereof.

However, when extraction is carried out using such a low polarity solvent, N-tert-butoxycarbonyl-L-tert-leucine can be hardly recovered at room temperature as 20 to 30°C and the temperature has to be raised to more than 40°C for recovery with good yield. As the result of the investigation by the present inventors, it was found that N-tert-butaxycarbonyl-L-tert-leucine is easily decomposed by heating and the decomposition starts at about 100°C. In general, thermal efficiency becomes worse in an industrial scale; therefore, thermal affection to a compound becomes strong. Since heating treatment is required in the above extraction and recovery, the extraction and recovery is not suitable for industrial production of N-tert-butoxycarbonyl-L-tert-leucine.

Furthermore, as the result of the study about the extraction procedure, the present inventors found that there are at least two crystal polymorphs of N-tert-butoxycarbonyl-L-tert-leucine. Until then, the crystal polymorph of N-tert-butoxycarbonyl-L-tert-leucine was not known, and the control technique of crystal polymorph was not known either. The stability, form (apparent condition), solubility, hygroscopic property, content of impurity and others change depending on a crystal form; and therefore, a crystal polymorph is very important factor in handleability and quality control of a crystal. In particular, the systemic absorbability of a medicine varies depending on a crystal form in some cases. Controlling the crystal polymorph of a compound is therefore very important not only in medicine field but also in stable industrial production of the compound.

MEANS FOR SOLVING THE PROBLEMS

The present inventors extensively studied the above problems; as a result, found that the use amount of an N-alcoxycarbonylating agent closely relates to the production of impurity in the production process of an N-alcoxycarbonyl-tert-leucine. In addition, the present inventors found that the use amount effect of an N-alcoxycarbonylating agent is exerted when the pH range is maintained in the range of not less than 9 and not more than 13 using a basic agent. In other words, the present inventors found that a high quality N-alcoxycarbonyl-tert-leucine can be easily produced with high yield by reducing the use amount of an N-alcoxycarbonylating agent to equimolar amount to be needed and maintaining the pH in the range of not less than 9 and not more than 13 using a basic pH adjuster.

The present invention relates to a process for production of an N-alcoxycarbonyl-tert-leucine,
comprising the step of reacting tert-leucine with an N-alcoxycarbonylating agent in the presence of water,
wherein the N-alcoxycarbonylating agent is not less than 0.90 times by mole and not more than 1.00 times by mole relative to the tert-leucine, and
the pH of a reaction mixture is maintained in the range of not less than 9 and not more than 13 using a basic agent.

In addition, surprisingly, the present inventors found that the extraction efficiency of an N-alcoxycarbonyl-tert-leucine can be dramatically improved by mixing a highly water-soluble hydroxide represented by the general formula (1):

$$ROH \qquad (1)$$

when an N-alcoxycarbonyl-tert-leucine is extracted from the basic aqueous solution of an N-alcoxycarbonyl-tert-leucine using a water-immiscible solvent; and as a result, an N-alcoxycarbonyl-tert-leucine can be efficiently recovered under a mild condition. Hereinafter, the above hydroxide is referred to as "the hydroxide (1)" in some cases.

Furthermore, the present inventors found that the crystal form of an N-alcoxycarbonyl-tert-leucine can be controlled by adjusting the water amount in crystallization procedure. In particular, the present inventor found that there are at least two types of crystal polymorph of N-tert-butoxycarbonyl-tert-leucine and the crystal polymorph can be controlled. The crystal polymorphs are referred to as Crystal A and Crystal B.

The present invention also relates to a process for production of an N-alcoxycarbonyl-L-tert-leucine, comprising the steps of mixing the basic aqueous solution of an N-alcoxycarbonyl-L-tert-leucine with at least one of a hydroxide represented by the following formula (1):

$$ROH \qquad (1)$$

wherein R is an optionally substituted $C_{1-6}$ alkyl group, in the amount of not less than 0.01 times by weight and not more than 5 times by weight relative to the N-alcoxycarbonyl-L-tert-leucine,
and then adding an acid and a water-immiscible solvent to the solution, to extract the N-alcoxycarbonyl-L-tert-leucine by the water-immiscible solvent.

The present invention also relates to a crystal of N-tert-butoxycarbonyl-tert-leuoine, wherein the crystal exhibits an Cu-K$\alpha$ x-ray powder diffraction pattern having specific peaks at 11.0°, 12.6°, 15.1°, 16.3°, 13.6°, 19.4°, 21.3° and 22.2° in degrees $2\theta \pm 0.1$; and a crystal of N-tert-butoxycarbonyl-tert-leucine, wherein the crystal exhibits an Cu-K$\alpha$ x-ray powder diffraction pattern having specific peaks at 8.6°, 14.4°, 15.9°, 17.3°, 19.0°, 21.9° and 22.3°in degrees $2\theta \pm 0.1$. The former crystal is Crystal A, and the latter crystal is Crystal B.

EFFECT OF THE INVENTION

According to the present invention method, a high quality N-alcoxycarbonyl-tert-leucine can be produced with high yield by easy procedure. The present invention method is therefore suitable for an industry production. In addition, according to the present invention method, an N-alcoxycarbonyl-L-tert-leucine can be efficiently extracted from the solution thereof under a mild condition; therefore, an N-alcoxycarbonyl-L-tert-leucine can be efficiently obtained in an industrial production without heat decomposition. Furthermore, the desired crystal form of an N-alcoxycarbonyl-L-tert-leucine having crystal polymorph, especially N-tert-butoxycarbonyl-L-tert-leucine, can be stably produced in an industrial process.

BRIER DESCRIPTION OF DRAWINGS

Figure 1 is a typical X-ray powder diffraction data of Crastal A.

Figure 2 is a typical X-ray powder diffraction data of Crastal B.

MODE FOR CARRYING OUT THE INVENTION

The first present invention is described. According to the first present invention method, a high quality N-alcoxycarbonyl-tert-leucine can be easily produced with high yield.
A commercially available L-tert-leucine may be used, and the L-tert-leucine prepared by a known method may be also used. For example, the synthesis reaction reported in JP10-72419A and the bio-reaction reported in JP9-504304T may be applied. In any methods, the type of tert-leucine is not particularly limited, and the crystal thereof and the solution thereof, such as an aqueous solution, may be used.
The optical purity of tert-leucine used in the present invention is not particularly limited. An optically active form, a racemic form, and the mixture of L-form and D-form in any mixing ratio may be used. According to the present invention method, the optical purity of the obtained N-alcoxycarbonyl-tert-leucine is generally maintained.
The reaction for N-alcoxycarbonylating L-tert-leucine is not particularly limited as long as the reaction is carried out in the presence of water. As the solvent, any one of water only, the mixed solvent of water and a water-sniscible solvent, and the two-layer solvent of water and a water-immiscible solvent can be preferably used.
An organic solvent is not particularly limited, and is exemplified by an aliphatic hydrocarbon solvent such as pentane, hexane, heptane and octane; an aromatic hydrocarbon solvent such as benzene, toluene and xylene; a halogen solvent such as methylene chloride, chlorobenzene, chloroform and 1,1,1-trichloroethane; an ether solvent such as tetrahydrofuran, 1,4-dioxane, diethyl ether, methyl tert-butyl ether and dibutyl ether; an ester solvent such as ethyl acetate, n-propyl acetate, isopropyl acetate, n-butyl acetate and tert-butyl acetate; a ketone solvent such as acetone, methyl ethyl ketone and methyl isobutyl ketone; an alcohol solvent such as methanol, ethanol, propanol and butanol; a nitrile solvent such as acetonitrile; an amide solvent such as N,N-dimethylformamide, N,N-dimethylacetamide and N-methylpyrrolidone; dimethylsulfoxide and others.
An organic solvent and water may be used alone, and alternatively, two or more solvents including water may be mixed in any mixing ratio and used. The order of mixing solvents is not also particularly limited. For example, as the solvent for L-tert-leucine, which is an amino acid, water and/or a water-miscible organic solvent is preferred for preferable reactivity and flowability of the reaction mixture even when a small amount of solvent is used, since the solubility of L-tert-leucine is high in water and a water-miscible solvent. In case of using an alcoxycarbonylating agent of which lipid solubility is high, such as di-tert-butyl dicarbonate, more preferable reactivity can be achieved when an organic solvent, especially a water-miscible organic solvent, is used than water only is used.
Among the exemplified solvents, a water-miscible solvent is exemplified by tetrahydrofuran, 1,4-dioxane, acetone, methanol, ethanol, n-propanol, isopropanol, n-butanol, isobutanol, tert-butanol and acetonitrile. In terms of general versatility and low toxicity, acetone, methanol, ethanol, isopropanol and tert-butanol are preferable.
The amount of a solvent to be used is not particularly limited, and tert-leucine may be completely dissolved or may not be completely dissolved at the starting point of the reaction. It is not necessarily needed to use a solvent enough to completely dissolve the produced N-alcoxycarbonyl-tert-leucine. The amount of a solvent relative to tert-leucine is preferably not less than 1.0 times by weight and not more than 50.0 times by weight, more preferably not less than 1.0 times by weight and not more than 20.0 times by weight, even more preferably not less than 2.0 times by weight and not more than 20.0 times by weight, and particularly preferably not less than 2.0 times by weight and not more than 15.0 times by weight, for preferable reactivity and flowability of the reaction mixture.
An inorganic salt may be contained as a coexisting material in the reaction mixture other than an organic solvent. The inorganic salt which may be contained is exemplified by sodium chloride, ammonium chloride, potassium chloride, sodium sulfate, ammonium sulfate, ammonium bromide and sodium hydrogencarbonate. However, an inorganic salt is not limited to the above examples. It can be easily determined by a simple experiment whether a certain inorganic salt can be contained or not.

The reaction temperature is not limited, and may be generally determined between the freezing point and the boiling point of the reaction mixture. The temperature is preferably not less than -20°C and not more than 90°, more preferably not less than -10°C and not more than 50°C, and even more preferably not less than 0°C and not more than 30°C.

The boiling point of a solution generally depends on a pressure. The reaction may be carried out under any conditions of ordinary pressure, reduced pressure and increased pressure. An appropriate pressure can be easily determined by a simple experiment.

The N-alcoxycarbonylating agent to be used in the reaction is not limited as long as the amino group of an amino acid can be N-alcoxycarbonylated by the agent. The agent is preferably a chloroformic acid alkyl ester or a dialkyl dicarbonate, more preferably a chloroformic acid alkyl ester and a dialkyl dicarbonate of which alkyl groups have not less than 1 and not more than 10 carbon atoms. In the present invention, an aralkyl group is included in the range of an alkyl group. An N-alcoxycarbonylating agent is specifically exemplified by methyl chloroformate, ethyl chloroformate, benzyl chloroformate, di-tert-butyl dicarbonate and dimethyl dicarbonate. An N-alcoxycarbonylating agent may be directly used, or once dissolved in an organic solvent and the solution may be used.

Needless to say, the structure of the used N-alcoxycarbonylating agent is reflected in the alcoxycarbonyl group of an N-alcoxycarbonyl-tert-leucine. The alkyl group of an alcoxycarbonyl group has not less than 1 and not more than 10 carbon atoms, and is specifically exemplified by a methyl group, an ethyl group, an n-propyl group, an isopropyl group, an n-butyl group, an isobutyl group, a tert-butyl group, a cyclopentyl group, a cyclohexyl group, a cyclohexyl group, a phenyl group, a benzyl group and a naphthyl group; more preferably a methyl group, an ethyl group, an isopropyl group, a tert-butyl group, a cyclopentyl group and a benzyl group; and particularly preferably a methyl group, an ethyl group, a tert-butyl group and a benzyl group.

It is very important in the present invention that the amount of an N-alcoxycarbonylating agent to be used is reduced to an equimolar of tert-leucine and the pH is maintained within the range of not less than 9 and not more than 13 for producing a highly pure N-alcoxycarbonyl-tert-leucine with high yield. Hereinafter, the point is specifically described.

In the present method, the pH during the reaction is maintained within the range of not less than 9 and not more than 13. If the pH becomes less than 9, the reactivity of tert-leucine markedly becomes poor and the reaction cannot be completed. In addition, even when the use amount of N-alcoxycarbonylating agent is reduced, a side reaction significantly progresses, as a result, the yield and quality of an N-alcoxycarbonyl-tert-leucine become lowered. On the other hand, even if the pH maintained at not less than 9, when the pH is beyond 13, the reaction cannot be similarly completed due to a severe decomposition of an N-alcoxycarbonylating agent. The pH is preferably not less than 9 and not more than 11, since the product can be obtained more easily in the pH range. In an industrial production, it is recommendable to control a pH in the range of not less than 10 and not more than 11 approximately. Under the condition of the present invention, even if the pH falls outside the range, quality and yield are unaffected and a stable production is possible.

The procedure for adjusting and maintaining the pH in the above-described range is not limited. In general, the pH which decreases with the progress of the reaction may be adjusted in the above-described range on a timely basis using a basic agent. Alternatively, in the case where the reaction can be completed in the above-described pH range, the pH at the start of the reaction may be set to be high in the above-described range and adjusted if necessary. With respect to the reagents, the order of addition, the addition rate, and the additive condition such as continuous addition and intermittent addition are not particularly limited as long as the pH can be maintained.

As the basic agent used for pH control, an inorganic base or a tertiary amine can be used, and an inorganic base is preferably used in terms of general versatility.

An inorganic base which can be used in the present invention is exemplified by an alkali metal hydroxide such as sodium hydroxide and potassium hydroxide; an alkali metal carbonate such as sodium carbonate and potassium carbonate; an alkali metal hydrogencarbonate such as sodium hydrogencarbonate and potassium hydrogencarbonate. An inorganic base is preferably an alkali metal hydroxide such as sodium hydroxide and potassium hydroxide; and an alkali metal carbonate such as sodium carbonate and potassium carbonate, in terms of strong basicity. A tertiary amine is exemplified by triethylamine, triisoprolyoamine and pyridine.

A basic agent may be directly used, or once dissolved in water or an organic solvent and the solution may be used. For promptly dispersing the added base into the reaction mixture, it is preferable that the solution of the base is used and the solid basic agent is dissolved in water or an organic solvent to be used. Needless to say, it is also possible that a liquid basic agent is dissolved in water or an organic solvent to be used.

It is preferable with respect to chemical property that the organic solvent used for dissolving a basic agent does not react with a basic agent and has poor responsiveness to tert-leucine and an N-alcoxycarbonylating agent under a basic condition. The physical property thereof, such as a boiling point and a melting point, is not particularly limited. It can be easily determined by a simple experiment whether a certain solvent can be used or not. A solvent to be used is exemplified by acetonitrile, acetone, tetrahydrofuran and toluene.

When a basic agent is used as a solution of water or an organic solvent, the concentration of the solution is not particularly limited but is preferably high in terms of production efficiency. In particular, sodium hydroxide, potassium hydroxide and potassium carbonate are easily dissolved in water, and the aqueous solution thereof can be used in high concentration. In such a case, the concentration is preferably not less than 30% by weight and not more than 50% by weight.

The procedure for adding a basic agent to maintain the pH is not limited. For example, the reaction may be completed with maintaining the pH in the above-mentioned range by adding a basic agent on a timely basis from the start of adding an N-alcoxycarbonylating agent, or an N-alcoxycarbonylating agent and a basic agent may be added alternately or at the same time.

The use amount of an N-alcoxycarbonylating agent has also to be controlled. Hereinafter, the amount is described.

In the present invention method, the use amount of an N-alcoxycarbonylating agent relative to tert-leucine for the reaction is preferably not less than 0.90 times by mole and not more than 1.00 times by mole, and more preferably not less than 0.95 times by mole and not more than 0.99 times by mole. If excessive amount of an N-alcoxycarbonylating agent relative to tert-leucine is used, it becomes easy that a side reaction proceeds and yield and quality of an N-alkoxy-tert-leucine are decreased even when the pH is maintained in the range of not less than 9 and not more than 13. In addition, it becomes easy that excessive amount of an N-alcoxycarbonylating agent and the decomposed compound of an N-alcoxycarbonylating agent is encontained in the target compound. On the other hand, if the use amount of an N-alcoxycarbonylating agent is much smaller than the necessary quantity, the yield would be decreased although the impurity derived from the agent is suppressed. Such a decreased yield results in the contamination of the unreacted tert-leucine and quality loss.

The addition rate of an N-alcoxycarbonylating agent is not limited as long as the pH can be controlled. The duration of the addition is preferably not more than 50 hours, and more preferably not less than 1 hour and not more than 20 hours.

In the reaction, the stirring speed may be adjusted to the extent that an N-alcoxycarbonylating agent and a basic agent are not locally inhomogeneous.

The duration time of stirring after the addition of an N-alcoxycarbonylating agent is not limited as long as the reaction is sufficiently completed, and is preferably not more than 50 hours and more preferably not less than 1 hour and not more than 20 hours in terms of production efficiency.

The thus obtained reaction mixture containing an N-alkoxy-tert-leucine may be subjected to an extraction procedure using an organic solvent under an appropriate pH for removing an inorganic salt, tert-leacine and a water-soluble organic compound.

In addition, the thus obtained reaction mixture or the extract may be subjected to a publicly known crystallization procedure to obtain a highly pure crystal of an N-alkoxy-tert-leucine. For example, the procedure to isolate the crystal can be carried out by extracting the produced N-alkoxy-tert-leucine into an aromatic hydrocarbon solvent with heating under an acidic condition, and then concentrating and cooling the extract for crystallization.

Next, the second present invention is described. The second present invention is a process for efficiently extracting an N-alcoxycarbonyl-L-tert-leucine from the solution thereof, specifically a basic aqueous solution of an N-alcoxycarbonyl-L-tert-leucine, under a mild condition.

First, a basic aqueous solution of an N-alcoxycarbonyl-L-tert-leucine used in the present invention is described.

In the basic aqueous solution of an N-alcoxycarbonyl-L-tert-leucine, an N-alcoxycarbonyl-L-tert-leucine, which is a carboxylic acid, forms a salt with a base under a basic condition and is dissolved.

It is preferable to obtain the basic aqueous solution of an N-alcoxycarbonyl-L-tert-leucine by the above-described process; however, a process for producing the solution is not limited, and the solution may be obtained by a general process for alcoxycarbonylating an amino acid. For example, such a general process is described in "Jikken Kagaku Kouza 5th edition (The Fifth Series of Experimental Chemistry)" published by MARUZEN PUBLISHING CO., LTD, vol. 16, p.224.

The pH during a general N-alcoxycarbonylating reaction is preferably basic, and the lower limit thereof is 7. The pH is preferably not less than 7.5 and more preferably not less than 8.0. The upper limit is not limited, and is preferably 13.0, more preferably 12.0, and particularly preferably 11.0, since it becomes easy under a strong alkaline condition that a side reaction such as racemization and the decomposition of a by-product are caused. The pH may be outside the above range as long as the reaction is not negatively affected. For example, the pH may fall outside the above range for a very short time.

The amount of an N-alcoxycarbonylating agent in a general N-alcoxycarbonylating reaction is not particularly limited. However, needless to say, the yield is decreased when the amount of an N-alcoxycarbonylating agent is too small relative to L-tert-leucine, and too much amount is not preferable since an impurity after the reaction is increased by a side reaction due to an excessive N-alcoxycarbonylating agent and a substance included in the agent. The use amount of an N-alcoxycarbonylating agent relative to L-tert-leucine is preferably not less than 0.9 times by mole and not more than 3 times by mole, more preferably 0.95 times by mole and not more than 2 times by mole, and even more preferably not less than 0.95 times by mole and not more than 1.5 times by mole, for a good production efficiency.

The pH of the thus obtained reaction mixture is basic in the above range. An N-alcoxycarbonyl-L-tert-leucine, which is a carboxylic acid, is dissolved as a salt with the base to be used. When an inorganic salt exists in water solvent, the layer of an N-alcoxycarbonyl-L-tert-leucine salt may be separated from the solution due to salting-out effect and the reaction mixture may be separated into two layers in some cases. In the present invention, even when the reaction mixture is separated into two layers, the total amount of the reaction mixture can be used in the extraction procedure. Alternatively, when the reaction mixture is separated into two layers, only the solution layer containing an N-alcoxycarbonyl-L-tert-leucine can be preferably used. In addition, the following solutions can be also preferably used:

- the solution produced by dissolving the separately purified N-alcoxycarbonyl-L-tert-leucine as a salt with the above base in water or a mixed solvent consisting water and the above water-miscible solvent;
- the basic aqueous solution obtained by extracting an N-alcoxycarbonyl-L-tert-leucine and then re-extracting the compound in water or a mixed solvent consisting water and the above water-miscible solvent.

Next, the process for extracting an N-alcoxycarbonyl-L-tert-leucine from the basic aqueous solution of an N-alcoxycarbonyl-h-tert-leucine using a water-immiscible solvent is described.

In general, the basic aqueous solution of an N-alcoxycarbonyl-L-tert-leucine is acidified by adding an acid, and then a water-immiscible solvent is added thereto for extraction procedure. The extraction procedure can be carried out under a mild condition without heating by mixing a hydroxide represented by the following formula (1):

$$ROH \qquad (1).$$

In the hydroxide (1), R is an optionally substituted $C_{1-6}$ alkyl group.

A $C_{1-6}$ alkyl group is not limited, and may be straight, branched, cyclic or acyclic. A $C_{1-6}$ alkyl group is exemplified by a methyl group, an ethyl group, an n-propyl group, an i-propyl group, an n-butyl group, an i-butyl group, a t-butyl group, an n-pentyl group, a cyclopentyl group, an n-hexyl group, a cyclohexyl group and others. The substituent thereof is exemplified by a halogen atom, a hydroxy group, an amino group, a carboxy group, an ether group and others.

Among the above examples, R is preferably a $C_{1-4}$ alkyl group, more preferably a methyl group, an ethyl group, an n-propel group, an i-propyl group, an n-butyl group, an i-butyl group and a t-butyl group, and even more preferably a methyl group, an ethyl group and an i-propyl group.

The hydroxide (1) may be used alone, and alternatively, two or more hydroxides (1) may be used in combination.

The amount of the hydroxide (1) relative to an N-alcoxycarbonyl-h-tert-leucine is not less than 0.01 times by weight and not more than 5 times by weight, preferably not less than 0.1 times by weight and not more than 4 times by weight, more preferably not less than 0.3 times by weight and not more than 3 times by weight, and particularly preferably not less than 0.4 times by weight and not more than 2 times by weight. When two or more hydroxides (1) are used, the amount means a total amount. When the solvent used in the reaction for obtaining an N-alcoxycarbonyl-L-tert-leucine is the hydroxide (1), the total amount containing the hydroxide (1) used as a solvent is adjusted so that the total amount is included in the above range.

Even a small amount of the hydroxide (1) can sufficiently accomplish a purpose. However, it is preferable to use a large amount of the hydroxide (1) for obtain Crystal B by crystallization as described later, since the water amount in the extract is increased.

A water-immiscible solvent is exemplified by an aliphatic hydrocarbon solvent such as pentane, hexane, cyclohexane, methylcyclohexane, heptane and octane; an aromatic hydrocarbon solvent such as benzene, toluene and xylene; a halogen solvent such as methylene chloride, chlorobenzene, chloroform and 1,1,1-trichloroethane; an ether solvent such as diethyl ether, methyl tert-butyl ether and dibutyl ether; an ester solvent such as ethyl acetate, n-propyl acetate, isopropyl acetate, n-butyl acetate and tert-butyl acetate; a ketone solvent such as methyl isobutyl ketone; and an alcohol solvent such as butanol. Among the examples, pentane, hexane, heptane, cyclohexane, methylcyclohexane, toluene, ethyl acetate, isopropyl acetate and methyl isobutyl ketone are preferable in terms of general versatility.

The amount of a water-immiscible solvent relative to an N-alcoxycarbonyl-L-tert-leucine is not less than 0.1 times by weight and not more than 30 times by weight, preferably not less than 0.5 times by weight and not more than 20 times by weight, and more preferably not less than 1 times by weight and not more than 10 times by weight. A water-immiscible solvent may be used alone, and alternatively, two or more solvents may be mixed in combination to be used.

The acid used for acidification is not limited, and may be an organic acid or a mineral acid. An organic acid is exemplified by trichloroacetic acid, trifluoroacetic acid, methansulfonic acid and p-toluenesulfonic acid. A mineral acid is exemplified by hydrochloric acid, sulfuric acid and phosphoric acid and nitric acid. In terms of general versatility and economic efficiency, a mineral acid is preferable, and hydrochloric acid and sulfuric acid are particularly preferable among a mineral acid.

The temperature during the extraction is not preferably high in terms of the present invention objective, and is preferably not less than -20°C and not more than 50°C, more preferably not less than -10°C and not more than 40°C, and even more preferably not less than 0°C and not more than 35°C.

The extraction procedure is not particularly limited. For example, the basic aqueous solution is acidified and then a water-immiscible solvent and the hydroxide (1) may be added thereto for extraction, or a water-immiscible solvent and the hydroxide (1) may be added and then the mixture may be acidified. The hydroxide (1) may be used in the reaction step. It is more preferable that the hydroxide (1) is used in the reaction step when water is used alone as a solvent, since the reaction may proceed more smoothly.

The solution containing a water-immiscible solvent obtained by the extraction procedure may be directly used in crystallization step. Hereinafter, the solution is referred to as "extract". If necessary, the extract may be washed using water for removing a water-soluble by-product. In addition, the target compound may be dissolved into a water layer again by basifying the extract for removing a by-product.

Next, the third present invention is described. According to the third present invention method, an N-alcoxycarbonyl-L-tert-leucine is isolated from the extract.

An N-alcoxycarbonyl-L-tert-leucine can be crystallized to be isolated from the extract by a general crystallization procedure. Such a crystallization procedure is exemplified by crystallization by cooling, crystallization by concentration and crystallization by adding a poor solvent. The crystal form can be controlled by adjusting the amount of water in the extract during the crystallization procedure. Specifically, an N-tert-butoxycarbonyl-L-tert-leucine has at least two crystal polymorphs, Crystal A and Crystal B; and the crystal polymorph can be controlled by adjusting the amount of water in the extract during the crystallization procedure. The "extract" from which an N-alcoxycarbonyl-L-tert-leucine is isolated may be obtained by the above extraction procedure or prepared by dissolving a preliminarily produced crystal in a solvent. When such a solution is used as the extract, the form of a crystal to be used is not limited.

The method for adjusting the water amount in the extract is not limited. The water amount can be adjusted by an easy procedure such as concentration and addition.

A concentration method is not limited, and can be preferably carried out under a necessary pressure. The temperature is not limited as long as an N-alcoxycarbonyl-L-tert-leucine does not decompose at the temperature. For example, the temperature is not less than -20°C and not more than 90°C, preferably not less than -10°C and not more than 70°C, and more preferably not less than 0°C and not more than 60°C.

The amount of water is adjusted to less than 0.1 w/w% or not less than 0.1 w/w% relative to an N-alcoxycarbonyl-L-tert-leucine. When the water amount is adjusted to less than 0.1 w/w% relative to an N-alcoxycarbonyl-L-tert-leucine, Crystal A may be obtained; and when the amount is adjusted to not less than 0.1 w/w% relative to an N-alcoxycarbonyl-L-tert-leucine, Crystal B may be obtained.

The Crystal A of N-tert-butoxycarbonyl-L-tert-leucine exhibits a Cu-Kα x-ray powder diffraction pattern having specific peaks at 11.0°, 12.6°, 15.1°, 16.3°, 18.6°, 19.4°, 21.3° and 22.2° in degrees 2θ ± 0.1. The dried Crystal A is stable at an ordinary temperature and an ordinary pressure, and does not change into the other crystal form (Crystal B) as long as a reasonable care such as avoiding direct sunlight, high temperature and high humidity is given to the Crystal A, even when the Crystal A is preserved for long periods. In addition, the Crystal A has the advantage that the Crystal A is more difficult to be dissolved in an organic solvent and easier to be recovered by crystallization than Crystal B. The amount of water to obtain Crystal A relative to N-tert-butoxycarbonyl-L-tert-leucine is less than 0.1 w/w%, preferably not more than 0.05 w/w%, and more preferably not more than 0.02 w/w%.

The Crystal B of N-tert-butoxycarbonyl-L-tert-leucine exhibits a Cu-Kα x-ray powder diffraction pattern having specific peaks at 8.6°, 14.4°, 15.9°, 17.3°, 19.0°, 21.9° and 22.3° in degrees 2θ ± 0.1. The dried Crystal B is stable at an ordinary temperature and a pressure, and does not change into the other crystal form (Crystal A) as long as a reasonable care such as avoiding direct sunlight, high temperature and high humidity is given to the Crystal B, even when the Crystal B is preserved for long periods. The crystal B has the advantage of handling property. For example, the concentration of Crystal B solution can be increased, since Crystal B is easier to be dissolved in an organic solvent than Crystal A. The amount of water to obtain Crystal B relative to N-tert-butoxycarbonyl-L-tert-leucine is not less than 0.1 w/w%, preferably not less than 0.5 w/w%, and more preferably not less than 1.0 w/w%.

An N-alcoxycarbonyl-L-tert-leucine can be crystallized from the above-described solution by a general crystallization procedure. Such a crystallization procedure is exemplified by making the concentration of an N-alcoxycarbonyl-L-tert-leucine in the solution to be oversaturated with a general procedure. Such a procedure is exemplified by cooling, concentration and adding a poor solvent. The cooling rate when the crystallization is carried out by cooling is not limited, and is preferably not less than 1°C/hour and not more than 50°C/hour, and more preferably not less than 5°C/hour and not more than 30°C/hour in terms of work efficiency. A poor solvent used for the crystallization is not particularly limited as long as the N-alcoxycarbonyl-L-tert-leucine solubility in the solvent is sufficiently low, and pentane, hexane, heptane, octane, cyclohexane, methylcyclohexane and others are preferably used.

According to the above procedure, an N-alcoxycarbonyl-L-tert-leucine can be spontaneously crystallized. However, if necessary, a seed crystal having the desired form may be added to the oversaturated solution of an N-alcoxycarbonyl-L-tert-leucine for crystallizing.

As the means for further decreasing the solubility in order to obtain the crystal of an N-alcoxycarbonyl-L-tert-leucine with good yield, the solution may be concentrated after crystallization, the solution may be further cooled, a poor solvent may be added to the solution, and two or more means may be carried out in combination.

The concentration of the N-alcoxycarbonyl-L-tert-leucine solution when crystallization is carried out is not limited, and may be appropriately adjusted depending on the used solvent and the crystallization method. The concentration of an N-alcoxycarbonyl-L-tert-leucine may be not less than 1 wt% and not more than 85 wt%, preferably not less than 5 wt% and not more than 80 wt%, and more preferably not less than 10 wt% and not more than 70 wt%, in terms of productivity and the flowability of the solution.

Each crystal form can be obtained by adjusting the amount of water in any crystallization method. To efficiently obtain Crystal A, crystallization by cooling and crystallization by adding a poor solvent are preferably applied in combination; and to efficiently obtain Crystal B, crystallization by neutralization described as follows is preferably applied.

The above-described crystallization by neutralization which is one means to obtain Crystal B is described.

In crystallization by neutralization, an N-alcoxycarbonyl-L-tert-leucine which is dissolved in water as a salt with a base is generally crystallized by neutralization using an acid. According to crystallization by neutralization, a crystal can be obtained very easily from a reaction mixture containing water by neutralization. Therefore, the means is economically and industrially advantageous. In the crystallization means, the amount of water is large and Crystal B can be therefore obtained. The solution of an N-alcoxycarbonyl-L-tert-leucine used for crystallization may be the reaction mixture, or prepared by once extracting the compound from the reaction mixture and then dissolving the compound into a water layer under a basic condition. Alternatively, the solution may be prepared by forming a salt of the preliminarily-obtained crystal with a base and dissolving the salt in water. In such a case, the crystal form of the preliminarily-obtained crystal is not limited.

A base used for crystallization by neutralization is not particularly limited, and may be one used in the reaction. A base is exemplified by an alkali metal hydroxide such as sodium hydroxide and potassium hydroxide; an alkaline-earth metal hydroxide such as magnesium hydroxide and calcium hydroxide; an alkali metal carbonate such as sodium carbonate and potassium carbonate; an alkali metal hydrogencarbonate such as sodium hydrogencarbonate and potassium hydrogencarbonate; an organic base such as pyridine and triethylamine; and others. Among the examples, in terms of cost and handleability, an inorganic base is preferable, and an alkali metal hydroxide, an alkali metal carbonate and an alkali metal hydrogencarbonate are more preferable. The acid used for neutralization is not similarly limited, and a versatile acid such as hydrochloric acid and sulfuric acid may be added for acidifying the solution.

A slurry containing a crystal of an N-alcoxycarbonyl-L-tert-leucine crystallized by the above means may be subjected to a general solid-liquid separation means such as centrifugation, filtration under an increased pressure and filtration under reduced pressure for isolating the crystal. The obtained crystal may be further dried under reduced pressure or in vacuo, if it is necessary to obtain a dry crystal.

The two crystal forms of the present invention are not necessarily pure respectively, and one crystal form may contain the other crystal form. In such a case, the ratio of the contaminant may be not more than 30%, preferably not more than 20%, and even more preferably not more than 10%.

An alcoxycarbonyl group is an N-protective group. An L-tert-leucine, which can be used as a raw material in the present invention, can be obtained by appropriately deprotecting an N-alcoxycarbonyl-L-tert-leucine. By repeating the protective reaction and deprotective reaction, much pure L-tert-leucine can be obtained.

As a typical deprotective reaction, for example, a tert-butoxycarbonylated compound is subjected to an acidic condition, a methoxycarbonylated compound is subjected to an basic condition, and a benzyloxycarbonylated compound is subjected to a hydrogenation reaction. The above-described extract containing an N-alcoxycarbonyl-L-tert-leucine may be subjected to a deprotective reaction. Alternatively, the isolated crystal having any crystal form may be subjected to a deprotective reaction.

The solvent used for the above deprotective reaction is preferably an organic solvent in which an N-alcoxycarbonyl-L-tert-leucine can be dissolved. For example, the solvent used for the above-described extraction may be used.

For example, the acid used for removing a tert-butoxycarbonyl group under an acidic condition is not particularly limited as long as the acid shows sufficient acid strength, and may be an organic acid or a mineral acid. An organic acid is exemplified by trichloroacetic acid, trifluoroacetic acid, methansulfonic acid and p-toluenesulfonic acid. A mineral acid is exemplified by hydrochloric acid, sulfuric acid, phosphoric acid and nitric acid. A mineral acid is preferable in terms of general versatility and economic efficiency, and is particularly preferably hydrochloric acid and sulfuric acid.

The order of addition and the addition rate are not particularly limited. For example, an acid may be added into the solution of an N-alcoxycarbonyl-L-tert-leucine, or the solution of an N-alcoxycarbonyl-L-tert-leucine may be added into an acid.

The reaction temperature is not limited as long as the used solvent and acid do not boil at the temperature. The reaction temperature is generally not less than 0°C and not more than 90°, preferably not less than 10°C and not more than 80°C, and particularly preferably not less than 15°C and not more than 70°C.

After the reaction, L-tert-leucine and an acid form a salt. In general, the salt is precipitated as a solid from an organic solvent such as a water-immiscible solvent, and dissolved in water and/or a water-miscible solvent. When an organic solvent is used and L-tert-leucine is obtained as a salt with an acid, the precipitated solid may be directly separated to obtain the target compound. When water and/or a water-miscible solvent is used, L-tert-leucine can be obtained as a salt with an acid by adjusting the ratio of the used water-miscible solvent or the temperature. In order to obtain only L-tert-leucine, in other words, to obtain L-tert-leucine without a counter ion, the reaction mixture is neutralized by adding a base thereto. A base is not limited as long as the base does not initiate a side reaction. The base which can be used in the present invention is exemplified by an alkali metal hydroxide such as sodium hydroxide and potassium hydroxide; an alkaline-earth metal hydroxide such as magnesium hydroxide and calcium hydroxide; an alkali metal carbonate such as sodium carbonate and potassium carbonate; an alkali metal hydrogencarbonate such as sodium hydrogencarbonate and potassium hydrogencarbonate; an organic base such as pyridine and tr-ethylamine.

A crystal can be obtained from the slurry containing a salt with an acid or the slurry obtained by neutralization using a general solid-liquid separation procedure such as centrifugation, filtration under increased pressure and filtration under reduced pressure. The thus obtained crystal may be dried by drying under reduced pressure or in vacuo, if needed.

EXAMPLES

Hereafter, the examples of the present invention are described, but the present invention is not limited by the examples. In the examples, the amount of the N-alcoxycarbonyl-L-tert-leucine contained in the solution is determined by HPLC analysis of the following condition.

HPLC condition

Column: Waters Symmetry C18 (3.5 $\mu$m, 150 mm X 4.6 mm i.d.)
Column temperature: 35°C
Detection device: UV detector (wavelength: 210 nm)
Mobile phase: the program shown in the following Table 1 was applied. In the Table 1, mobile phase A is a 0.1% by weight phosphoric acid aqueous solution and mobile phase B is acetonitrile.
Flow rate: 1.0 mL/min

Table 1

| Ratio of mobile phases A and B for HPLC | | |
|---|---|---|
| Time (min) | Mobile phase A | Mobile phase B |
| 0 | 95 | 5 |
| 25 | 5 | 95 |
| 30 | 5 | 95 |
| 30.1 | 95 | 5 |
| 45 | STOP | |

The amount of L-tert-leucine was measured with the following analysis condition.

Column: CAPCELLPAKSCX (250 mm X 4.6 mm i.d.)
Mobile phase: solution prepared by mixing a phosphate buffer (pH = 3.3) and acetonitrile in the volume ratio of 95 : 5
Flow rate: 1.0 mL/min
Column temperature: 35°C
Detection device: Differential refractive index detector

The yield in the examples was calculated by the following formula.

```
Yield (%) = (the total amount of the produced N-
alcoxycarbonyl-L-tert-leucine) × 100 / (the total amount of
the used L-tert-leucine)
```

The reaction selectivity means the ratio of the obtained N-alcoxycarbonyl-L-tert-leucine relative to the L-tert-leucine used in the reaction. When the reaction selectivity is high, the impurity derived from tert-leucine is suppressed. The reaction selectivity was calculated by the following formula.

Reaction selectivity (%) = (yield) × 100 / [(total amount of the used L-tert-leucine)-(amount of the remaining L-tert-leucine)]

Example 1

A 15% by weight sodium hydroxide aqueous solution was added to the aqueous solution (72.4 g) containing L-tert-leucine (13.2 g, 0.10 mol), to adjust the pH to 13.0. Then, methyl chloroformate (9.52 g, 0.10 mol, 1.00 equivalent) was slowly added thereto with maintaining the temperature of the mixture below 25°C. At the time, the pH was decreased by adding methyl chloroformate; however, the pH of the solution was maintained at 10.0-12.8 by simultaneously adding a 15% by weight of sodium hydroxide aqueous solution. After the addition of methyl chloroformate, the mixture was stirred for 2 hours. Then, the yield and quality were analyzed by HPLC. Yield: 98%, Reaction selectivity: 100%

Example 2

An aqueous solution (303.37 g) containing L-tert-leucine (30.03 g, 0.23 mole) was cooled to 10°C. Methyl chloroformate (21.63 g, 0.23 mol, 1.00 equivalent) was slowly added thereto with maintaining the pH of the mixture at 9.0-9.5 using a 30% by weight of sodium hydroxide aqueous solution. After the addition, the mixture was stirred at 10°C for 12 hours. Then, the yield and the amount of the generated by-product were analyzed by HPLC.
Yield: 97%, Reaction selectivity: 99%

Examples 3 to 6

The reaction was carried out in the same condition as Example 2 except that the pH was adjusted to 9.5-10.0, 10.0-10.5, 10.5-11.0 or 9.0-13.0.
Table 2

| Example No. | pH of reaction mixture | Yield | Reaction selectivity ratio |
|---|---|---|---|
| 3 | 9.5-10.0 | 98% | 99% |
| 4 | 10.0-10.5 | 98% | 99% |
| 5 | 10.5-11.0 | 98% | 99% |
| 6 | 9.0-13.0 | 98% | 99% |

Example 7

The reaction was carried out in the same condition as Example 6 except that an aqueous solution of L-tert-leucine was cooled to 20°C.
Yield: 97%, Reaction selectivity: 99%

Example 8

The reaction was carried out in the same condition as Example 6 except that D-tert-leucine was used instead of L-tert-leucine.

The D-tert-leucine was analyzed as in the case of L-tert-leucine.
Yield: 97%, Reaction selectivity: 99%

Example 9

The reaction was carried out in the same condition as Example 6 except that racemic tert-leucine was used instead of L-tert-leucine.
The racemic tert-leucine was analyzed as in the case of L-tert-leucine.
Yield: 97%, Reaction selectivity: 98%

Comparative Example 1

In a 15% by mass of sodium hydroxide aqueous solution (191.7 g), L-tert-leucine (85.0 g, 0.65mol) was dissolved. Methyl chloroformate (68.9 g, 0.73 mol, 1.13 equivalents) was added dropwise thereto over 1 hour with maintaining the temperature between 5°C and 15°C. At the time, the pH of the reaction mixture was kept at 9.0-9.5 by appropriately adding a 15% by mass of sodium hydroxide aqueous solution for the reaction. After the addition of methyl chloroformate, the reaction mixture was stirred at 20°C for 1 hour.
Yield: 90%, Reaction selectivity: 90%

Comparative Example 2

The reaction was carried out in the same condition as Comparative Example 1 except that the pH was adjusted to 10.0-10.5.
Yield: 93%, Reaction selectivity: 93%

Comparative Example 3

The reaction was carried out in the same condition as Comparative Example 1 except that the pH was adjusted to 13.5-14.0.
Yield: 82%, Reaction selectivity: 97%

Comparative Example 4

Dioxane (9.1 ml) was added to an aqueous solution (29.3 g) containing L-tert-leucine (2.18 g, 0.017 mol) and sodium hydroxide (2.35 g, 0.059 mol), and then, methyl chloroformate (1.57 g, 0.017 mol, 1.00 equivalent) was slowly added thereto at room temperature. After the addition, the reaction mixture was heated to 60°C and stirred for 14 hours. The obtained solution was analyzed by HPLC; as a result, the amount of N-methoxycarbonyl-tert-leucine to be produced was only 1.0 g. The pH of the obtained solution was 13.9 at 30°C, and the pH at the start of the reaction was beyond 14.
Yield: 33%, Reaction selectivity: 63%

Example 10

An aqueous solution (101.06 g) containing L-tert-leucine (10.36 g, 0.079 mol) was cooled to 5°C. Benzyl chloroformate (13.49 g, 0.079 mol, 1.00 equivalent) was slowly added with maintaining the pH between 9.0 and 9.5 using a 48% by weight of sodium hydroxide aqueous solution. After the addition, the mixture was stirred at 20°C for 14 hours. Then, the yield and the amount of the generated impurity were analyzed by HPLC.
Yield: 96%, Reaction selectivity: 97%

Examples 11 and 12

The reaction was carried out in the same condition as Example 10 except that the pH was maintained in 10.0-10.5 or 9.0-13.0.
Table 3

| Example No. | pH of reaction mixture | Yield | Reaction selectivity ratio |
|---|---|---|---|
| 11 | 10.0-10.5 | 97% | 98% |
| 12 | 9.0-13.0 | 97% | 97% |

Example 13

The reaction was carried out in the same condition as Example 12 except that the aqueous solution containing L-tert-leucine was cooled to 20°C.
Yield: 97%, Reaction selectivity: 98%

Example 14

The reaction was carried out in the same condition as Example 12 except that D-tert-leucine was used instead of L-tert-leucine.
Yield: 97%, Reaction selectivity: 98%

Example 15

The reaction was carried out in the same condition as Example 12 except that racemic tert-leucine was used instead of L-tert-leucine.
Yield: 97%, Reaction selectivity: 99%

Comparative Example 5

The reaction was carried out in the same condition as Comparative Example 1 except that benzyl chloroformate was used instead of methyl chloroformate.
Yield: 90%, Reaction selectivity: 90%

Comparative Example 6

The reaction was carried out in the same condition as Comparative Example 5 except that the pH of the reaction mixture was maintained in 10.0-10.5.
Yield: 94%, Reaction selectivity: 94%

Comparative Example 7

The reaction was carried out in the same condition as Example 10 except that the pH of the reaction mixture was maintained in not less than 14.0.
Yield: 86%, Reaction selectivity: 95%

Example 16

The reaction was carried out in the same condition as Example 6 except that ethyl chloroformate was used instead of methyl chloroformate.
Yield: 97%, Reaction selectivity: 98%

Example 17

An aqueous solution (250.5 g) containing L-tert-leucine (20.5 g, 0.16 mol) was cooled to 7°C, and a 48% by weight of sodium hydroxide aqueous solution (16.5 g) was added thereto. Then, di-tert-butyl dicarbonate (34.92 g, 0.16 mol, 1.00 equivalent) was slowly added with maintaining the pH in 9.4-10.8. After the addition, the mixture was stirred at not more than 20°C for 14 hours. Then, the yield and the amount of the generated impurity were analyzed by HPLC.
Yield: 96%, Reaction selectivity: 98%

Example 18

The reaction was carried out in the same condition as Example 17 except that D-tert-leucine was used instead of L-tert-leucine.
Yield: 97%, Reaction selectivity: 97%

Example 19

The reaction was carried out in the same condition as Example 17 except that racemic tert-leucine was used instead of L-tert-leucine.
Yield: 96%, Reaction selectivity: 98%

Comparative Example 8

The reaction was carried out in the same condition as Comparative Example 1 except that di-tert-butyl dicarbonate was used instead of methyl chloroformate and the temperature was maintained between 20°C and 30°C.
Yield: 89%, Reaction selectivity: 89%

Comparative Example 9

The reaction was carried out in the same condition as Comparative Example 8 except that the pH of the reaction mixture was maintained between 10.0 and 10.5.
Yield: 92%, Reaction selectivity: 92%

Example 20

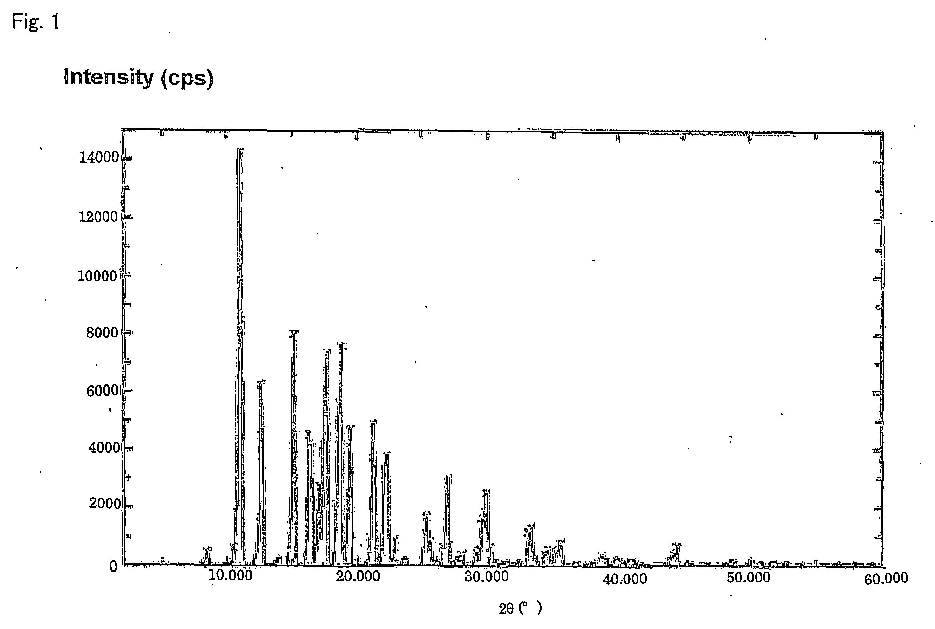

An aqueous solution (1150 g) containing L-tert-leucine (100 g, 762 mmol) was stirred, and the pH of the solution was adjusted to 9 using a 30% by weight of sodium hydroxide aqueous solution. Then, isopropanol (160 g) was added thereto. Further, di-tert-butyl dicarbonate (174.7 g, 800 mmol) was added thereto at 25°C with maintaining the pH between 8.5 and 9.5 using a 30% by weight of sodium hydroxide aqueous solution. The mixture was stirred for about 3 hours, and the resultant was analyzed; as a result, 175 g of N-tert-butoxycarbonyl-L-tert-leucine was produced (yield: 99%).
Toluene (600 g) was added thereto at 25°C, and the pH was adjusted to 3 using 35% by weight of hydrochloric acid. The organic layer separated at the same temperature was analyzed; as a result, it was found that 174 g of the compound (1) (N-tert-butoxycarbonyl-tert-leucine) was contained (extraction yield: 99%). The effect of the present invention could be confirmed as the result, compared with the description of Patent Document 3 that the compound (1) (N-tert-butoxycarbonyl-tert-leucine) can be hardly recovered using an aromatic hydrocarbon solvent at about room temperature of 20 to 30°C.
The above extract was washed with water (300 g) twice, and then, concentrated until the amount became about 300 g. The procedure that toluene (200 g) was added and the mixture was concentrated depending on the additive amount was repeated twice, and the amount of the mixture was finally adjusted to 270 g. The water amount ratio relative to N-tert-butoxycarbonyl-L-tert-leucine in the final mixture was 0.01 w/w%.
To the obtained slurry at 45°C, n-hexane (570 g) was added. The mixture was cooled to 0°C and maintained at the same temperature for 1 hour. Then, the crystal was separated and the solvent was removed, to obtain a white crystal of N-tert-butoxycarbonyl-L-tert-leucine (161 g, total yield: 91%).
It was confirmed that the obtained crystal was Crystal A by powder X-ray diffraction measurement (Cu-K$\alpha$) (Fig. 1). Main diffraction angle in powder X-ray diffraction pattern (Cu-K$\alpha$) (2θ $\pm$ 0.1): 11.0°, 12. 6°, 15.1°, 16.3°, 18.6°, 19.4°, 21.3° and 22.2°

Example 21

Crystal B (6.17 g) was dissolved in toluene (12.33 g) and tert-butanol (0.02 g) at 50°C, and n-hexane (25 g) was added thereto for crystallization. The water amount relative to crystal B was 0.005 w/w%. The mixture was cooled to room temperature, and 4.94 g of the crystal was obtained by separation and drying (yield: 90%).
It was confirmed that the obtained crystal was Crystal A by powder X-ray diffraction measurement (Gu-K$\alpha$).

Example 22

The Crystal A of N-tert-butoxycarbonyl-L-tert-leucine (2.15 g) was dissolved in a solvent consisting of methylcyclohexane (12 g) and tert-butanol (0.46 g) and water (0.44 g). The solution was concentrated until the amount became 3.4 g. Methylcyclohexane (21 g) was added thereto, and the mixture was concentrated until the amount became 2.8 g. Methylcyclohexane (21 g) was added thereto, to obtain a slurry. The water concentration was 0.14% by weight in the slurry, and was 1.5 w/w% relative to Crystal A. The crystal was obtained by separation and drying (1.83 g, yield: 80%).

It was confirmed that the obtained crystal was Crystal B by powder X-ray diffraction measurement (Cu-K$\alpha$).

Example 23

The Crystal A of N-tert-butoxycarbonyl-L-tert-leucine (1.44 g) was dissolved by adding tert-butanol (0.03 g), water (0.03 g) and toluene (2 g) at 50°C. To the solution, n-hexane (6 g) was added. The water amount in the mixture was 2.1 w/w% relative to the compound (1) (N-tert-butoxycarbonyl-tert-leucine). Crystallization was stimulated by adding 1 mg of Crystal B. After the mixture was cooled to room temperature, a crystal was obtained by separation (1.00 g, yield: 69%).

It was confirmed that the obtained crystal was Crystal B by powder X-ray diffraction measurement (Cu-K$\alpha$).

Example 24

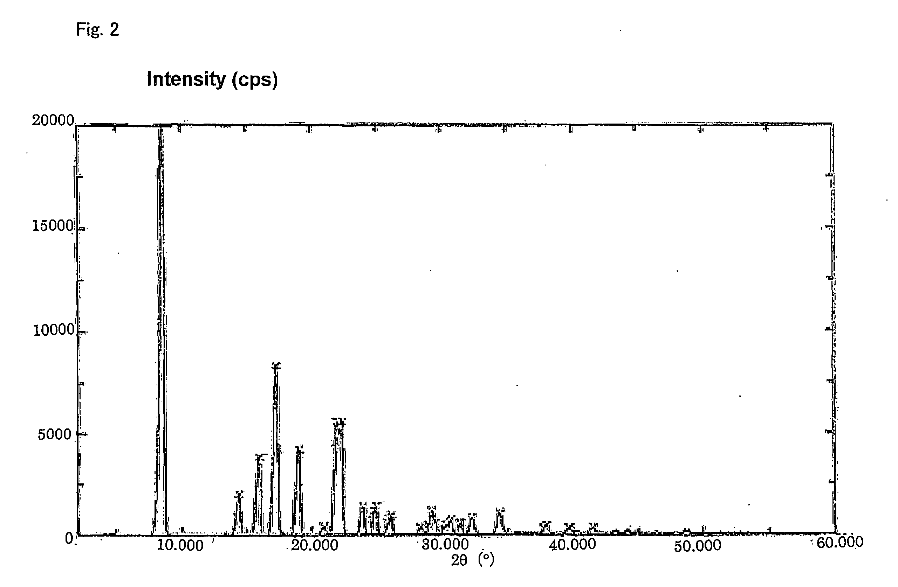

Crystal A of N-tert-butoxycarbonyl-L-tert-leucine (2.5 g) was dispersed in water (15.4 g), and dissolved by adding a 30% by weight of sodium hydroxide aqueous solution (1.8 g). To the mixture, 35% by weight of hydrochloric acid (1.4 g) was added with cooling by ice. As a result, a crystal was precipitated during the addition. The water amount was 703 w/w% relative to the compound (1) (N-tert-butoxycarbonyl-tert-leucine). After the mixture was stirred for 1 hour, the crystal was separated and dried (2.30 g, yield: 92%). It was confirmed that the obtained crystal was Crystal B by powder X-ray diffraction measurement (Cu-K$\alpha$) (Fig. 2).

Main diffraction angle in powder X-ray diffraction pattern (Cu-K$\alpha$) ($2\theta \pm 0.1$): 8.6°, 14.4°, 15.9°, 17.3°, 19.0°, 21.9° and 22.3°

Example 25

Crystal A and Crystal B of N-tert-butoxycarbonyl-L-tert-leucine were respectively dispersed in a mixed solvent of toluene / n-hexane = 1 / 5 (w/w). After 4 hours, the slurries were filtered, and the mother liquid concentrations were compared with each other. The mother liquid concentration in case of Crystal A was 0.4% by weight, and was 0.8% by weight in case of Crystal B. The mother liquid concentration of the slurry in which Crystal B was dispersed after 72 hours was 0.4% by weight. The crystal obtained after 72 hours was Crystal A according to the results of powder X-ray diffraction measurement (Cu-K$\alpha$).

Reference Example

Crystal A of N-tert-butoxycarbonyl-L-tert-leucine (100 g, 432 mmol) was dissolved in toluene (400 g). To the solution, 35% hydrochloric acid (54 g, 519 mmol) was added at room temperature. After the mixture was stirred in a hot-water bath at 60°C for 3 hours, the water layer was obtained by separating the organic layer. A solution consisting of methanol (80 g) and triethylamine (52.6 g, 520 mmol) was kept in a hot-water bath at 60°C, and the water layer was slowly added thereto to obtain a slurry. Then, the mixture was cooled using a ice bath for 1 hour. The crystal of L-tert-leucine was obtained by separation and drying (52 g, yield: 92%).

**Claims**

1.    A process for production of an N-alcoxycarbonyl-tert-leucine,
      comprising the step of reacting tert-leucine with an N-alcoxycarbonylating agent in the presence of water,
      wherein the N-alcoxycarbonylating agent is not less than 0.90 times by mole and not more than 1.00 times by mole relative to the tert-leucine, and
      the pH of a reaction mixture is maintained in the range of not less than 9 and not more than 13 using a basic agent.

2.    The production process according to claim 1, wherein the pH of the reaction mixture of the tert-leucine and the N-alcoxycarbonylating agent is not less than 9 and not more than 11.

3.    The production process according to claim 1 or 2, wherein the N-alcoxycarbonylating agent is a chloroformic acid alkyl ester or a dialkyl dicarbonate.

4. The production process according to claim 3, wherein the N-alcoxycarbonylating agent is a chloroformic acid alkyl ester or a dialkyl dicarbonate, and the alkyl group thereof has not less than 1 and not more than 10 carbon atoms.

5. The production process according to claim 4, wherein the N-alcoxycarbonylating agent is methyl chloroformate, ethyl chloroformate, benzyl chloroformate or di-tert-butyl dicarbonate.

6. The production process according to any one of claims 1 to 5, wherein the basic agent used for maintaining the pH of the reaction mixture is sodium hydroxide, potassium hydroxide, sodium carbonate or potassium carbonate.

7. The production process according to claim 6, wherein the basic agent is an aqueous solution having a concentration of not less than 30% by weight and not more than 50% by weight.

8. A process for production of an N-alcoxycarbonyl-L-tert-leucine, comprising the steps of
reacting tert-leucine with an N-alcoxycarbonylating agent to obtain a basic aqueous solution of an N-alcoxycarbonyl-tert-leucine,
mixing the basic aqueous solution with not less than 0.01 times by weight and not more than 5 times by weight of at least one of a hydroxide represented by the following formula (2):

$$ROH \qquad (2)$$

wherein R is an optionally substituted $C_{1-6}$ alkyl group,
and then adding an acid and a water-immiscible solvent to extract the N-alcoxycarbonyl-L-tert-leucine by the water-immiscible solvent.

9. The production process according to claim 8, wherein the N-alcoxycarbonyl-tert-leucine is N-tert-butoxycarbonyl-L--tert-leucine.

10. The production process according to claim 8 or 9, wherein the water-immiscible solvent is at least one of pentane, hexane, heptane, cyclohexane, methylcyclohexane, toluene, ethyl acetate, isopropyl acetate and methyl isobutyl ketone.

11. The production process according to any one of claims 8 to 10, wherein the amount of water in the extract is adjusted to less than 0.1 w/w% relative to the N-alcoxycarbonyl-tert-leucine, and then crystallization is carried out.

12. The production process according to any one of claims 8 to 10, wherein the amount of water in the extract is adjusted to not less than 0.1 w/w% relative to the N-alcoxycarbonyl-tert-leucine, and then crystallization is carried out.

13. A crystal of N-tert-butoxycarbonyl-tert-leucine, wherein the crystal exhibits an Cu-K$\alpha$ x-ray powder diffraction pattern having specific peaks at 11.0°, 12.6°, 15.1°, 16.3°, 18.6°, 19.4°, 21.3° and 22.2° in degrees 26 $\pm$ 0.1.

14. A crystal of N-tert-butoxycarbanyl-tert-leucine, wherein the crystal exhibits an Cu-K$\alpha$ x-ray powder diffraction pattern having specific peaks at 8.6°, 14.4°, 15.9°, 17.3°, 19.0°, 21.9° and 22.3° in degrees 28 $\pm$ 0.1.

Fig. 1

Fig. 2

**Intensity (cps)**

EP 2 423 187 A1

<table>
<tr><td colspan="2"><b>INTERNATIONAL SEARCH REPORT</b></td><td>International application No.<br>PCT/JP2009/062687</td></tr>
</table>

A. CLASSIFICATION OF SUBJECT MATTER
C07C269/04(2006.01)i, C07C271/12(2006.01)i

According to International Patent Classification (IPC) or to both national classification and IPC

B. FIELDS SEARCHED

Minimum documentation searched (classification system followed by classification symbols)
C07C269/04, C07C271/12

Documentation searched other than minimum documentation to the extent that such documents are included in the fields searched
Jitsuyo Shinan Koho        1922–1996    Jitsuyo Shinan Toroku Koho   1996–2009
Kokai Jitsuyo Shinan Koho  1971–2009    Toroku Jitsuyo Shinan Koho   1994–2009

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2008-125364 A  (Mitsubishi Rayon Co., Ltd.),<br>05 June 2008 (05.06.2008),<br>example 23<br>(Family: none) | 13<br>1-12,14 |
| A | JP 2004-175703 A  (Mitsubishi Rayon Co., Ltd.),<br>24 June 2004 (24.06.2004),<br>claims; examples<br>(Family: none) | 1-14 |
| A | JP 2003-146962 A  (Mitsubishi Rayon Co., Ltd.),<br>21 May 2003 (21.05.2003),<br>claims; examples<br>(Family: none) | 1-14 |

☒ Further documents are listed in the continuation of Box C.    ☐ See patent family annex.

| | |
|---|---|
| *    Special categories of cited documents:<br>"A"  document defining the general state of the art which is not considered   to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>06 October, 2009 (06.10.09) | Date of mailing of the international search report<br>20 October, 2009 (20.10.09) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| | International application No. |
|---|---|
| | PCT/JP2009/062687 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2005-119974 A  (Sumitomo Chemical Co., Ltd.), 12 May 2005 (12.05.2005), claims; examples (Family: none) | 1-14 |
| A | JP 2007-131589 A  (Mitsubishi Rayon Co., Ltd.), 31 May 2007 (31.05.2007), claims; referential examples; examples (Family: none) | 1-14 |
| A | JP 2007-238495 A  (Mitsubishi Rayon Co., Ltd.), 20 September 2007 (20.09.2007), claims; preparation examples; examples (Family: none) | 1-14 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009008913 A
- WO 2009005677 A
- JP 2004175703 A
- JP 2001501216 T
- JP 2003146962 A
- JP 2008125364 A
- JP 10072419 A
- JP 9504304 T

**Non-patent literature cited in the description**

- Jikken Kagaku Kouza 5th edition. MARUZEN PUB-LISHING CO., LTD, vol. 16, 224